Europäisches Patentamt

European Patent Office

Office européen des brevets

(11) Publication number: **0 030 870**

**B1**

(12) EUROPEAN PATENT SPECIFICATION

(45) Date of publication of patent specification: **20.03.85**

(21) Application number: **80304566.5**

(22) Date of filing: **17.12.80**

(51) Int. Cl.⁴: **C 07 D 203/08, C 07 D 203/02**

(54) Process for producing solutions of aziridine-2-carboxylic acid salt.

(30) Priority: **18.12.79 JP 163588/79**

(43) Date of publication of application:
**24.06.81 Bulletin 81/25**

(45) Publication of the grant of the patent:
**20.03.85 Bulletin 85/12**

(84) Designated Contracting States:
**CH DE FR GB IT LI NL**

(56) References cited:

**CHEMISCHE BERICHTE, vol. 93, no. 7, 1960, Weinheim/Bergstr., K.D. GUNDERMANN et al. "Bildung, Ringspaltung und Isomerisierung von Äthylenimin-carbonsäure-(2)-Derivaten", pages 1632 to 1643**

The file contains technical information submitted after the application was filed and not included in this specification

(73) Proprietor: **MITSUI TOATSU CHEMICALS, INCORPORATED**
**2-5 Kasumigaseki 3-chome**
**Chiyoda-Ku Tokyo 100 (JP)**

(72) Inventor: **Kawashima, Nobuyuki**
**4-1-28, Hase**
**Kamakura-shi Kanagawa-ken (JP)**
Inventor: **Higuchi, Chojiro**
**4-5-13, Dai**
**Kamakura-shi Kanagawa-ken (JP)**
Inventor: **Kato, Toshio**
**600-1, Kamisakunobe Takatsu-ku**
**Kawasaki-shi Kanagawa-ken (JP)**
Inventor: **Mita, Ryuichi**
**529, Shimosakunobe Takatsu-ku**
**Kawasaki-shi Kanagawa-ken (JP)**
Inventor: **Yamaguchi, Akihiro**
**1-1-21, Iwase**
**Kamakura-shi Kanagawa-ken (JP)**
Inventor: **Nagai, Shosuke**
**1071-2, Nakano-cho Totsuka-ku**
**Yokohama-shi Kanagawa-ken (JP)**
Inventor: **Takano, Takao**
**380-172, Watauchi**
**Fujisawa-shi Kanagawa-ken (JP)**

(56) References cited:

Chemical Abstracts, vol. 89, no. 13, 25 September 1978, Columbus, Ohio, USA, K. NAKAJIMA et al. "Studies on aziridine-2-carboxylic acid. I. Synthesis of the optically active L-aziridine-2-carboxylic acid and its derivatives", page 998, column 1, abstract no. 110314a

(74) Representative: Armitage, Ian Michael et al
MEWBURN ELLIS & CO. 2/3 Cursitor Street
London EC4A 1BQ (GB)

## Description

This invention relates to a novel process for producing solutions of an aziridine-2-carboxylic acid salt which salt is very useful as an intermediate for an alpha-amino acid, an agricultural chemical and a pharmaceutical.

Some methods for the production of an aziridine-2-carboxylic acid salt have been known. For example, for the production of sodium aziridine-2-carboxylate, there are known (1) a method which comprises neutralizing alpha-chloro-beta-alanine hydrochloride in water with an aqueous solution of sodium hydroxide, and while heating the neutralization product under reflux, adding a 1N aqueous solution of sodium hydroxide dropwise so that the pH of the aqueous solution is maintained at 7—7.5 [K. D. Gundermann, Chem. Ber., *93*, 1640 (1960)], and (2) a method which comprises hydrolyzing isopropyl aziridine-2-carboxylate obtained by reaction between isopropyl alpha,beta-dibromopropionate and liquid ammonia, with sodium ethoxide in a mixed solvent consisting of ether, ethanol and water [E. Kyburz, Helv. Chim. Acta. *49*, 368 (1966)]. Lithium aziridine-2-carboxylate is known to be produced by (3) a method which comprises treating alpha-chloro-beta-alanine ethyl ester hydrochloride with triethanolamine in ethanol to form ethyl aziridine-2-carboxylate, and hydrolyzing the product with lithium hydroxide in a mixed solvent consisting of ethanol and water [K. D. Gundermann, Chem. Ber., *93*, 1639 (1960)]. The method (1), however, has the defect that it has to be operated while the concentration of the starting alpha-chloro-beta-alanine hydrochloride in the reaction system is maintained at as low as about 1% by weight. The methods (2) and (3), on the other hand, have the defect that synthesis of the aziridine-2-carboxylic acid ester is complex and the yield of the final product is low. Accordingly, none of these prior methods are entirely satisfactory in commercial practice.

It is an object of this invention therefore to provide a commercially advantageous process for producing solutions of an aziridine-2-carboxylic acid salt.

The present invention provides a process for producing solutions of aziridine-2-carboxylic acid salt, characterised in that it comprises treating at a temperature of 20 to 80°C an alpha-halogeno-beta-aminopropionic acid, its $C_1$—$C_4$ alkyl ester or a mineral acid salt of said acid or ester with an alkali metal hydroxide, an alkaline earth metal hydroxide or aqueous ammonia in water or a mixture of water and a water miscible organic solvent, wherein:

(i) the amount of said water or mixture of water and a water miscible organic solvent is 3 to 100 times the amount of starting alpha-halogeno-beta-aminopropionic acid, its $C_{1-4}$ alkyl ester or a mineral acid salt of said acid or ester;

(ii) the amount of said alkali metal hydroxide, alkaline earth metal hydroxide or aqueous ammonia used is 2 to 5 equivalents based on the starting material when the starting material is an alpha-halogeno-beta-aminopropionic acid or its $C_1$—$C_4$ alkyl ester, or is 3 to 5 equivalents based on the starting material when the starting material is a mineral acid salt of said acid or ester; and

(iii) the aqueous ammonia or the alkali or alkaline earth metal hydroxide as an aqueous solution or a solid is added with stirring to a solution of the alpha-halogeno-beta-aminopropionic acid, its $C_{1-4}$ alkyl ester or a mineral acid salt of said acid or ester in water or in a mixture of water and water-miscible organic solvent in a desired concentration, or the alpha-halogeno-beta-aminopropionic acid, its $C_{1-4}$ alkyl ester or a mineral acid salt of said acid or ester is added to aqueous ammonia or an aqueous solution or suspension of the alkali or alkaline earth metal hydroxide.

An aziridine-2-carboxylic acid salt is a very important intermediate for synthesis of amino acids and other compounds, as is demonstrated, for example by the fact that treatment of lithium aziridine-2-carboxylate, in 15% sulfuric acid yields serine, an alpha-amino acid.

The process for producing solutions of aziridine-2-carboxylic acid salts in accordance with this invention has many advantages over the prior art in that it does not require pH control or heating and the reaction operation is simple, that an aziridine-2-carboxylic acid salt is produced by using the reactant in a higher concentration than in the prior art in the reaction solution. The specific examples given herein show the desired product being obtained selectively without substantially forming byproducts. Accordingly, the process of this invention is of very high utilitarian value in commercial practice.

In the process of this invention, the starting alpha-halogeno-beta-aminopropionic acid or its mineral acid salt may, for example, include alpha-chloro-beta-aminopropionic acid, alpha-bromo-beta-aminopropionic acid, alpha-chloro-beta-aminopropionic acid hydrochloride or sulfate, and alpha-bromo-beta-aminopropionic acid hydrochloride or sulfate. Examples of the alpha-halogeno-beta-aminopropionic acid ester or its mineral acid salt are alpha-chloro-beta-aminopropionic acid esters, alpha-bromo-beta-aminopropionic acid esters, alpha-chloro-beta-aminopropionic acid ester hydrochlorides and sulfates, and alpha-bromo-beta-aminopropionic acid ester hydrochlorides and sulfates. The ester used is an alkyl ester having 1 to 4 carbon atoms in the alkyl moiety, and methyl esters, ethyl esters, isopropyl esters and tertiary butyl esters are used frequently.

The mineral acid salt of an alpha-halogeno-beta-aminopropionic acid used as a starting material in the process of this invention is obtained by reacting an alpha,beta-dihalogenopropionitrile or an alpha-halogeno- acrylonitrile with ammonia in water or in an organic solvent, and causing hydrogen chloride or sulfuric acid to act on the reaction mixture. The alpha-halogeno-beta-aminopropionic acid can be isolated by dissolving its mineral acid salt in the smallest amount of water, adding anhydrous ethanol, and neutralizing it [K. D. Gundermann, Chem. Ber., *91*, 160 (1958)]. The mineral acid salt of an alpha-halogeno-beta-aminopropionic acid ester, for example, methyl alpha-chloro-beta-aminopropionate hydrochloride, can be obtained by esterifying alpha-chloro-beta-aminopropionic acid hydrochloride in anhydrous methanol saturated with hydrochloric acid [K. D. Gundermann, Chem. Ber., *91*, 160 (1958)].

In the process of this invention, an alkali metal hydroxide, an alkaline earth metal hydroxide, or aqueous ammonia is used to perform the cyclization reaction of an alpha-halogeno-beta-aminopropionic acid and the cyclization reaction and hydrolysis reaction of an alpha-halogeno-beta-aminopropionic acid ester. Specific examples of the alkali metal hydroxide or alkaline earth metal hydroxide are lithium hydroxide, sodium hydroxide, potassium hydroxide, beryllium hydroxide, magnesium hydroxide, calcium hydroxide and barium hydroxide. The alkali is used in an amount of 2 to 5 equivalents based on the starting material when the starting material is a free alpha-halogeno-beta-aminopropionic acid or an alpha-halogeno-beta-aminopropionic acid $C_{1-4}$ alkyl ester, and in an amount of 3 to 5 equivalents based on the starting material when the starting material is a mineral acid salt of the alpha-halogeno-beta-aminopropionic acid or the alpha-halogeno-beta-aminopropionic acid $C_{1-4}$ alkyl ester.

The process of this invention is performed in water or in a mixture of water and a water-miscible organic solvent. Thus, the reaction can be performed in an aqueous solution or in a solvent consisting of water and a water-miscible organic solvent. Examples of the water-miscible organic solvent include alcohols such as methanol, ethanol, n-propanol, isopropanol, tert.-butanol, Cellosolve and methyl Cellosolve, acetone, dioxane, tetrahydrofuran, N,N-dimethyl formamide, N,N-diethyl formamide and dimethyl sulfoxide. These organic solvents may be used as a mixture of two or more. When water and the organic solvent are used together, the ratio between them can be chosen as desired. The amount of water or the mixture of water and water-miscible organic solvent is 3 to 100 times, preferably 5 to 100 times, and especially (as illustrated in the examples herein) less than 12 times, the amount of the starting alpha-halogeno-beta-aminopropionic acid, its $C_{1-4}$ alkyl ester or a mineral acid salt of said acid or ester.

In performing the process of this invention, the method and order of adding the starting material, the alkali and the reaction solvent does not have to be regulated to control the pH. The aqueous ammonia or the alkali or alkaline earth metal hydroxide as an aqueous solution or a solid is added with stirring to a solution of the alpha-halogeno-beta-aminopropionic acid, its $C_{1-4}$ alkyl ester or a mineral acid salt of said acid or ester in water or in a mixture of water and a water-miscible organic solvent in a desired concentration. Alternatively, the alpha-halogeno-beta-aminopropionic acid, its $C_{1-4}$ alkyl ester or a mineral acid salt of the acid or ester may be added to aqueous ammonia or an aqueous solution or suspension of the alkali or alkaline earth metal hydroxide.

The reaction temperature is in the range of 20 to 80°C. The reaction is carried out usually under atmospheric pressure, but if desired, it may be carried out under reduced or elevated pressure. The reaction time varies depending upon the reaction conditions. Usually, it comes to an end in 0.5 to 50 hours, particularly in 2.0 to 30 hours. The end point of the reaction can be rapidly and easily determined by thin-layer chromatography.

In the process of this invention, the aziridine-2-carboxylic acid is obtained in the form of a solution of an alkali metal salt, an alkaline earth metal salt or an ammonium salt corresponding to the alkali metal hydroxide, alkaline earth metal hydroxide or aqueous ammonia used in the reaction. If desired, the organic solvent may be removed from the reaction product under reduced pressure to obtain the salt in the form of an aqueous solution.

The following Examples illustrate the process of this invention more specifically.

Lithium aziridine-2-carboxylate as a standard sample used in the Examples was synthesized in the following manner. [K. D. Gundermann, Chem. Ber. *93*, 1639 (1960)].

Production of lithium aziridine-2-carboxylate

10 g of ethyl alpha-chloro-beta-aminopropionate hydrochloride was dissolved in 100 ml of dehydrated ethanol, and 20 g of triethanolamine was added. With stirring, they were reacted at 60 to 70°C for 5 hours. The precipitated triethanolamine hydrochloride was separated by filtration, and washed with a small amount of ethanol. The filtrate and the washing were combined, and with cooling, 55 ml of a 1N aqueous solution of lithium hydroxide was gradually added. The mixture was allowed to stand for 24 hours in a cold dark place. The reaction mixture was then concentrated to dryness under reduced pressure. Then, 30 ml of benzene was added to the residue (syrupy material), and water was completely removed by azotropic distillation. Then, 50 ml of hot ethanol was added, and the mixture was cooled to form a precipitate. 100 ml of ether was added to precipitate crystals fully. The crystals were separated by filtration, and washed with

|  | C | H | N | Li |
|---|---|---|---|---|
| Found (%): | 37.86 | 4.23 | 14.71 | 7.40 |
| Calculated (%): | 38.74 | 4.33 | 15.06 | 7.46 |

The purity of the resulting lithium aziridine-2-carboxylate, determined by a proton NMR spectrum using dioxane as an internal standard (measuring solvent: $D_2O$, measuring temperature: room temperature), was 97%.

Conditions for analysis by high-speed liquid chromatography in the Examples were as follows.

Column: Shodex OH Pak B-804 (a product of Showa Denko K.K.).

Detector: Differential refractometer RI-2 (a product of Nippon Bunseki Kogyo Co., Ltd.).

Mobile phase: Aqueous solution of $H_3PO_4$ ($1 \times 10^{-3}$ mole/liter).

Flow rate: 1.3 ml/min.

Measuring temperature: room temperature.

Under these conditions, the retention time of aziridine-2-carboxylic acid was 16.5 to 16.8 minutes.

Example 1

24 g of alpha-chloro-beta-aminopropionic acid hydrochloride was dissolved in 70 g of water. With stirring, a solution of 20 g of sodium hydroxide in 50 g of water was added dropwise to the resulting solution. Then, the reaction was performed at room temperature for 24 hours. The reaction mixture was analyzed by high-speed liquid chromatography. It was found that the yield of sodium aziridine-2-carboxylate was 93.5% based on the alpha-chloro-beta-aminopropionic acid hydrochloride.

When the resulting reaction mixture was analyzed by proton NMR spectroscopy, a signal attributed to the methylene proton at $\delta=1.79$ ppm (q., 2H) and a signal attributed to the methine proton at $\delta=2.35$ ppm (q., 1H) were detected. This signal pattern was identical with that of lithium aziridine-2-carboxylate used as the standard sample.

Example 2

17.3 g of alpha-chloro-beta-aminopropionic acid sulfate was dissolved in 100 g of water, and a solution of 18.0 g of potassium hydroxide in 60 g of water was added dropwise to the resulting solution with stirring. The reaction was performed at room temperature for 30 minutes. The reaction mixture was analyzed by high-speed liquid chromatography. The yield of potassium aziridine-2-carboxylate formed was 92.3%.

Example 3

24 g of alpha-chloro-beta-aminopropionic

ether to afford 1.0 g of lithium aziridine-2-carboxylate.

Melting point: 260—268°C (decomp.)

Elemental analysis (%) for $C_3H_4NO_2Li$:

acid hydrochloride was dissolved in 100 g of water, and a solution of 13 g of lithium hydroxide in 50 g of water was added dropwise to the resulting solution with stirring. The reaction mixture was then heated to 60°C, and reacted at 60 to 65°C for 4 hours. The reaction mixture was analyzed by high-speed liquid chromatography. The yield of lithium aziridine-2-carboxylate formed was 90.8%.

Example 4

Example 3 was repeated except that 18.5 g of free alpha-chloro-beta-aminopropionic acid was used instead of the alpha-chloro-beta-aminopropionic acid hydrochloride, and 21 g of sodium hydroxide was used instead of the lithium hydroxide. The yield of sodium aziridine-2-carboxylate formed was 89.6%.

Example 5

10.2 g of alpha-bromo-beta-aminopropionic acid hydrochloride was dissolved in 80 g of water, and a solution of 7 g of sodium hydroxide in 40 g of water was added dropwise to the resulting solution with stirring. The reaction was then performed at room temperature for 24 hours. The yield of sodium aziridine-2-carboxylate formed was 87.5%.

Example 6

16.0 g of alpha-chloro-beta-aminopropionic acid hydrochloride was dissolved in a mixed solvent consisting of 90 g of water and 50 g of methanol, and an aqueous solution of 10.5 g of lithium hydroxide in 50 g of water was added dropwise to the resulting solution with stirring. Then, the reaction mixture was heated to 60°C, and reacted at 60 to 65°C for 8 hours. The methanol was distilled off under reduced pressure from the reaction mixture, and the residue was analyzed by high-speed liquid chromatography. The yield of lithium aziridine-2-carboxylate formed was 85.6%.

Example 7

Example 6 was repeated except that 50 g of isopropyl alcohol was used instead of methanol, and 13 g of sodium hydroxide was used instead of lithium hydroxide. The yield of sodium aziridine-2-carboxylate formed was 90.5%.

Example 8

17.4 g of methyl alpha-chloro-beta-aminopropionate hydrochloride was dissolved in 140 g of water, and a solution of 13 g of sodium hydroxide in 60 g of water was added dropwise to the resulting solution with stirring. The mixture was then reacted at room temperature for 30 hours. The yield of sodium aziridine-2-carboxylate was 84.9%.

Example 9

Example 8 was repeated except that 18.8 g of ethyl alpha-chloro-beta-aminopropionate was used instead of the methyl alpha-chloro-beta-aminopropionate hydrochloride. The yield of sodium aziridine-2-carboxylate formed was 83.2%.

Example 10

A solution of 19 g of alpha-chloro-beta-aminopropionic acid hydrochloride in 50 g of water was added dropwise to 150 g of 8% aqueous ammonia, and they were reacted at room temperature for 48 hours. The yield of ammonium aziridine-2-carboxylate formed was 85.9%.

The foregoing examples illustrate that as compared with references 2 and 3 high yields are obtainable from the process of the present invention under mild reaction conditions. As compared with reference 1 the reaction can be operated at reasonable concentrations.

**Claims**

1. A process for producing solutions of aziridine-2-carboxylic acid salt, characterised in that it comprises treating at a temperature of 20 to 80°C an alpha-halogeno-beta-aminopropionic acid, its $C_1$—$C_4$ alkyl ester or a mineral acid salt of said acid or ester with an alkali metal hydroxide, an alkaline earth metal hydroxide or aqueous ammonia in water or a mixture of water and a water-miscible organic solvent, wherein:

(i) the amount of said water or mixture of water and a water-miscible organic solvent is 3 to 100 times the amount of starting alpha-halogeno-beta-aminopropionic acid, its $C_{1-4}$ alkyl ester or a mineral acid salt of said acid or ester;

(ii) the amount of said alkali metal hydroxide, alkaline earth metal hydroxide or aqueous ammonia used is 2 to 5 equivalents based on the starting material when the starting material is an alpha-halogeno-beta-aminopropionic acid or its $C_1$—$C_4$ alkyl ester, or is 3 to 5 equivalents based on the starting material when the starting material is a mineral acid salt of said acid or ester; and

(iii) the aqueous ammonia or the alkali or alkaline earth metal hydroxide as an aqueous solution or a solid is added with stirring to a solution of the alpha-halogeno-beta-aminopropionic acid, its $C_{1-4}$ alkyl ester or a mineral acid salt of the said acid or ester in water or in a mixture of water and a water-miscible organic solvent in a desired concentration, or the alpha-halogeno-beta-aminopropionic acid, its $C_{1-4}$

alkyl ester or a mineral acid salt of said acid or ester is added to aqueous ammonia or an aqueous solution or suspension of the alkali or alkaline earth metal hydroxide.

2. The process of claim 1 wherein the amount of said water or solvent is 5 to 100 times the amount of said acid, ester or salt.

3. The process of claim 1 or claim 2 wherein the amount of said water or solvent is less than 12 times the amount of said acid, ester or salt.

4. The process of any one of claims 1, 2 and 3 wherein the alpha-halogeno-beta-aminopropionic acid is alpha-chloro-beta-aminopropionic acid or alpha-bromo-beta-aminopropionic acid.

5. The process of any one of claims 1, 2 and 3 wherein the alpha-halogeno-beta-aminopropionic acid ester is a methyl ester, ethyl ester, isopropyl ester or tert.-butyl ester of alpha-chloro, or alpha-bromo-beta-aminopropionic acid.

6. The process of any one of claims 1, 2 and 3 wherein the mineral acid salt is a sulfate or hydrochloride.

7. The process of any one of claims 1, 2 and 3 wherein the alkali metal hydroxide is sodium hydroxide, potassium hydroxide or lithium hydroxide.

8. The process of any one of claims 1, 2 and 3 wherein the alkaline earth metal hydroxide is calcium hydroxide.

9. The process of any one of claims 1, 2 and 3 wherein the water-miscible organic solvent is at least one solvent selected from methanol, ethanol, n-propanol, isopropanol, t-butanol, 2-ethoxyethanol, 2-methoxyethanol, acetone, dioxane, tetrahydrofuran, N,N-dimethyl formamide, N,N-diethyl formamide and dimethyl sulfoxide.

**Patentansprüche**

1. Verfahren zur Herstellung von Lösungen von Aziridin-2-carbonsäuresalz, dadurch gekennzeichnet, daß es das Behandeln einer $\alpha$-Halogen-$\beta$-aminopropionsäure, ihres $C_1$—$C_4$-Alkylesters oder eines Mineralsalzes der Säure oder des Esters mit einem Alkalimetallhydroxid, einem Erdalkalimetallhydroxid oder wässrigem Ammoniak in Wasser oder einem Gemisch aus Wasser und einem mit Wasser mischbaren organischen Lösungsmittel bei einer Temperatur von 20 bis 80°C umfaßt, wobei:

(i) die Menge des Wassers oder des Gemischs aus Wasser und einem mit Wasser mischbaren organischen Lösungsmittel das 3- bis 100-fache der Menge der Ausgangs-$\alpha$-Halogen-$\beta$-aminopropionsäure, ihres $C_1$-$C_4$-Alkylesters oder eines Mineralsalzes der Säure oder Esters ist,

(ii) die Menge des verwendeten Alkalimetallhydroxids, Erdalkalimetallhydroxids oder wässrigen Ammoniaks 2 bis 5 Äquivalente, bezogen auf das Ausgangsmaterial, wenn das

Ausgangsmaterial eine $\alpha$-Halogen-$\beta$-aminopropionsäure oder deren $C_1$—$C_4$-Alkylester ist, oder 3 bis 5 Äquivalente, bezogen auf das Ausgangsmaterial, wenn das Ausgangsmaterial ein Mineralsäuresalz der Säure oder des Esters ist, und

(iii) das wässrige Ammoniak oder das Alkali- oder Erdalkalimetallhydroxid als wässrige Lösung oder Feststoff unter Rühren zu einer Lösung der $\alpha$-Halogen-$\beta$-aminopropionsäure, ihres $C_1$—$C_4$-Alkylesters oder eines Mineralsäuresalzes der Säure oder des Esters in Wasser oder in einem Gemisch aus Wasser und einem mit Wasser mischbaren organischen Lösungsmittel in einer gewünschten Konzentration gegeben wird oder die $\alpha$-Halogen-$\beta$-aminopropionsäure, ihr $C_1$—$C_4$-Alkylester oder ein Mineralsäuresalz der Säure oder des Esters zu wässrigem Ammoniak oder einer wässrigen Lösung oder Suspension des Alkali- oder Erdalkalimetallhydroxids gegeben wird.

2. Verfahren nach Anspruch 1, worin die Menge des Wassers oder Lösungsmittels das 5- bis 100-fache der Menge der Säure, des Esters oder Salzes ist.

3. Verfahren nach Anspruch 1 oder Anspruch 2, worin die Menge des Wassers oder Lösungsmittels weniger als das 12-fache der Menge der Säure, des Esters oder Salzes ist.

4. Verfahren nach irgend einem der Ansprüche 1, 2 und 3, worin die $\alpha$-Halogen-$\beta$-aminopropionsäure $\alpha$-Chlor-$\beta$-aminopropionsäure oder $\alpha$-Brom-$\beta$-aminopropionsäure ist.

5. Verfahren nach irgend einem der Ansprüche 1, 2 und 3, worin der $\alpha$-Halogen-$\beta$-aminopropionsäureester ein Methylester, Ethylester, Isopropylester oder tert.-Butylester von $\alpha$-Chlor- oder $\alpha$-Brom-$\beta$-aminopropionsäure ist.

6. Verfahren nach irgend einem der Ansprüche 1, 2 und 3, worin das Mineralsäuresalz ein Sulfat oder Hydrochlorid ist.

7. Verfahren nach irgend einem der Ansprüche 1, 2 und 3, worin das Alkalimetallhydroxid Natriumhydroxid, Kaliumhydroxid oder Lithiumhydroxid ist.

8. Verfahren nach irgend einem der Ansprüche 1, 2 und 3, worin das Erdalkalimetallhydroxid Calciumhydroxid ist.

9. Verfahren nach irgend einem der Ansprüche 1, 2 und 3, worin das mit Wasser mischbare organische Lösungsmittel wenigstens ein Lösungsmittel, ausgewählt unter Methanol, Ethanol, n-Propanol, Isopropanol, t-Butanol, 2-Ethoxyethanol, 2-Methoxyethanol, Aceton, Dioxan, Tetrahydrofuran, N,N-Dimethylformamid, N,N-Diethylformamid und Dimethylsulfoxid, ist.

### Revendications

1. Procédé pour l'obtention de solutions de sel d'acide aziridine-2-carboxylique, caractérisé en ce qu'il comprend le traitement à une température de 20 à 80°C d'un acide alpha-halogéno-bêta-aminopropionique, un ester d'alcoyle en $C_1$—$C_4$ de celui-ci ou un sel d'acide minéral de cet acide ou ester avec un hydroxyde de métal alcalin, un hydroxyde de métal alcalino-terreux ou l'ammoniaque aqueuse dans l'eau ou dans un mélange d'eau et d'un solvant organique miscible dans l'eau, où:

(i) la quantité de l'eau ou du mélange d'eau et du solvant organique miscible dans l'eau est de 3 à 100 fois la quantité d'acide alpha-halogéno-bêta-aminopropionique de départ, d'un ester d'alcoyle en $C_1$—$C_4$ de celui-ci ou d'un sel d'acide minéral de cet acide ou ester;

(ii) la quantité dudit hydroxyde de métal alcalin, hydroxyde de métal alcalino-terreux ou ammoniaque aqueuse utilisée est de 2 à 5 équivalents basés sur le produit de départ lorsque le produit de départ est un acide alpha-halogéno-bêta-aminopropionique ou un ester d'alcoyle en $C_1$—$C_4$ de celui-ci ou est de 3 à 5 équivalents basés sur le produit de départ lorsque le produit de départ est un sel d'acide minéral dudit acide ou ester; et

(iii) l'ammonique aqueuse ou hydroxyde de métal alcalin ou alcalino-terreux en solution aqueuse ou solide est ajouté avec agitation à une solution de l'acide alpha-halogéno-bêta-aminopropionique, un ester d'alcoyle en $C_{1-4}$ de celui-ci ou un sel d'acide minéral de cet acide ou ester dans l'eau ou dans un mélange d'eau et d'un solvant organique miscible dans l'eau en une concentration désirée, ou l'acide alpha-halogéno-bêta-aminopropionique, un ester d'alcoyle en $C_{1-4}$ de celui-ci ou un sel d'acide minéral de cet acide ou ester est ajouté à l'ammoniaque aqueuse ou à une solution aqueuse ou suspension de l'hydroxyde de métal alcalin ou alcalino-terreux.

2. Procédé selon la revendication 1, où la quantité de ladite eau ou solvant est de 5 à 100 fois la quantité de cet acide, ester ou sel.

3. Procédé selon la revendication 1 ou 2, où la quantité de l'eau précitée ou du solvant est inférieure à 12 fois la quantité de l'acide, ester ou sel précité.

4. Procédé selon l'une des revendications 1, 2 ou 3, où l'acide alpha-halogéno-bêta-aminopropionique précité est l'acide alpha-chloro-bêta-aminopropionique ou l'acide alpha-bromo-bêta-aminopropionique.

5. Procédé selon l'une des revendications 1, 2 ou 3, où l'ester d'acide alpha-halogéno-bêta-aminopropionique est un méthylester, éthylester, isopropylester ou tert-butylester d'acide alpha-chloro- ou alpha-bromo-bêta-aminopropionique.

6. Procédé selon l'une des revendications 1, 2 ou 3, où le sel d'acide minéral précité est un sulfate ou hydrochlorate.

7. Procédé selon l'une des revendications 1, 2 ou 7, ou l'hydroxyde de métal alcalin est l'hydroxyde de sodium, l'hydroxyde de potassium ou l'hydroxyde de lithium.

8. Procédé selon l'une des revendications 1, 2 ou 3, où l'hydroxyde de métal alcalino-terreux est l'hydroxyde de calcium.

9. Procédé selon l'une des revendications 1, 2 ou 3, où le solvent organique miscible dans l'eau est au moins l'un des solvants choisis parmi méthanol, éthanol, n-propanol, isopropanol, t-butanol, 2-éthoxyéthanol, 2-méthoxyéthanol, acétone, dioxane, tétrahydro-furane, N,N-dimethyl formamide, N,N-diéthyl formamide et diméthyl sulfoxyde.